# EUROPEAN PATENT APPLICATION

(11) **EP 3 995 164 A1**
(43) Date of publication of application: **11.05.2022**
(21) Application number: 20837646.7
(22) Date of filing: 02.07.2020
(51) Int. Cl.: A61M 5/14, A61M 25/02, A61M 5/00, A61M 5/168, A61M 5/165

(54) **COVER FOR DRUG DELIVERY TUBE DEVICE AND DRUG DELIVERY TUBE DEVICE SET COMPRISING SAME**

(30) Priority: 05.07.2019 KR 20190081439
(71) Applicant: Kim, Yong Hyun, Goyang-si, Gyeonggi-do 10483 (KR)
(72) Inventor: Kim, Yong Hyun, Goyang-si, Gyeonggi-do 10483 (KR)
(74) Representative: Santarelli
(86) International application number: PCT/KR2020/008634
(87) International publication number: WO 2021/006536

(57) **Abstract**

The cover for a drug delivery tube device according to a disclosed embodiment is configured to define an insertion space having an opening formed on a lower side thereof so that the drug delivery tube device having a capillary flow path configured to allow a drug solution to flow therethrough is capable of being inserted into the opening. The cover includes a cover body extending along a circumference of the insertion space around a center axis perpendicular to an upper-lower direction.

## Description

### TECHNICAL FIELD

The present disclosure relates to a cover for a drug delivery tube device and a drug delivery tube device set including the same.

### BACKGROUND

In order to supply a drug to a patient, a drug injection device for injecting a liquid drug solution (e.g., an injection solution) to a patient is known. Using the drug injection device, the drug solution in a predetermined storage space is introduced into the body of the patient through a passage (e.g., the inner spaces of a tube and an injection needle) connected to the patient.

For medical purposes, there is known a device including a drug delivery tube configured to prevent a drug solution from being injected into the patient's body at one time and form a capillary flow path so that the drug solution is slowly injected into the body for a considerable period of time. By allowing the drug solution to pass through the drug delivery tube, an hourly flow rate of the drug solution flowing along the passage of the drug solution injection device is limited.

As the drug delivery tube thermally expands or contracts due to the temperature change of the surrounding environment of the patient, the cross-sectional area of the capillary flow path is changed according to the temperature change. As a result, there is a problem that the flow rate of the drug solution flowing along the capillary flow path is not constant and fluctuates by a considerable range. Embodiments of the present disclosure provide a device for reducing a range of change in a flow rate of a drug solution due to a change in temperature.

Conventionally, as a drug delivery tube device is used for a long time by attaching it to the patient's skin, there is a problem in that the patient feels discomfort in the skin. In particular, when the drug delivery tube device is attached to the skin for a long time while a portion of the drug delivery tube device applies a concentrated load to one point of the patient's skin, there is a problem in that the patient may feel pain in the skin. Embodiments of the present disclosure provide a device for reducing discomfort or pain of a patient when a drug delivery tube device is attached to the patient's skin.

### SUMMARY

One aspect of the present disclosure provides embodiments of a cover for a drug delivery tube device. The cover for the drug delivery tube device according to a representative embodiment is configured to define an insertion space having an opening formed on a lower side thereof so that a drug delivery tube device having a capillary flow path configured to allow a drug solution to flow therethrough can be inserted into the opening. The cover includes a cover body extending along a circumference of the insertion space around a center axis perpendicular to an upper-lower direction.

In one embodiment, the cover body may be configured to be elastically deformed so that the opening is capable of being widened left and right.

In one embodiment, the cover body may be formed of a flexible material containing silicon.

In one embodiment, a plurality of grooves recessed and spaced apart from one another may be formed on an inner surface of the cover body.

In one embodiment, the cover may further include a pair of bases disposed below the cover body and extending in a horizontal direction perpendicular to the upper-lower direction, each of the bases having a lower end surface which is a lowermost end of the cover. The lower end surfaces of the bases may be located at the same level in the upper-lower direction.

In one embodiment, the cover may further include an auxiliary base disposed below the cover body, the auxiliary base having a lower end surface spaced apart from the lower end surfaces of the pair of bases in an extension direction of the center axis and disposed at the same level in the upper-lower direction with the lower end surfaces of the pair of bases.

In one embodiment, the cover may further include a base disposed below the cover body and having a lower end surface which is a lowermost end of the cover. A ventilation groove recessed upward and extending to an edge of the lower end surface may be formed on the lower end surface.

In one embodiment, the ventilation groove may be spaced apart from the opening.

In one embodiment, the cover may further include an engaging portion extending from the cover body in a horizontal direction perpendicular to the upper-lower direction and forming at least one of a hook and a hole.

In one embodiment, the cover may further include an engaging portion extending from the cover body in a horizontal direction perpendicular to the upper-lower direction; a base disposed below the cover body, extending in the horizontal direction perpendicular to the upper-lower direction, and having a lower end surface which is a lowermost end of the cover; and an auxiliary base disposed below the cover body, spaced apart from the base with the engaging portion interposed between the auxiliary base and the base, and having a lower end surface disposed at the same level in the upper-lower direction as the lower end surface of the base.

In one embodiment, the engaging portion may have a lower end surface disposed at a higher level in the upper-lower direction than the lower end surface of the base.

In one embodiment, the insertion space may extend along the center axis.

In one embodiment, the insertion space may have an opening at at least one of both ends in an extension direction of the center axis.

In one embodiment, the cover body may include a capillary flow path accommodation portion configured to form a capillary flow path accommodation space in the insertion space to accommodate the capillary flow path therein, and a filter accommodation portion configured to form a filter accommodation space in the insertion space to accommodate an air passage filter of the drug delivery tube device therein.

In one embodiment, a flow path groove recessed and extending along the center axis may be formed on an inner surface of the filter accommodation portion.

Another aspect of the present disclosure provides embodiments of a drug delivery tube device set. The drug delivery tube device set according to a representative embodiment includes: a drug delivery tube device having a capillary flow path configured to allow a drug solution to flow therethrough; and a cover configured to define an insertion space having an opening formed on a lower side thereof so that the drug delivery tube device is capable of being inserted into the opening, the cover extending along a circumference of the insertion space around a center axis perpendicular to an upper-lower direction.

In one embodiment, the cover may include a base having a lower end surface disposed at the same position in the upper-lower direction as a lowermost end of the drug delivery tube device or at a position lower than the lowermost end of the drug delivery tube device in a state in which the drug delivery tube device is inserted into the insertion space.

In one embodiment, the cover may include an engaging portion extending in a horizontal direction perpendicular to the upper-lower direction. The drug delivery tube device set may further include a fixing member with which the engaging portion is engaged, and an attachment member to which the fixing member is fixed, the attachment member being configured to be attachable to a user.

In one embodiment, the drug delivery tube device may include a hydrophobic air passage filter for discharging an air in the drug solution to the outside. The cover may include a capillary flow path accommodation portion configured to form a capillary flow path accommodation space in the insertion space to accommodate the capillary flow path therein, and a filter accommodation portion configured to form a filter accommodation space in the insertion space to accommodate an air passage filter of the drug delivery tube device therein.

In one embodiment, the drug delivery tube device may have an inlet port formed at at least one of both ends in an extension direction of the center axis to allow the drug solution to flow into the inlet port. The insertion space may have an opening disposed at one, at which the inlet port is disposed, of both ends in the extension direction of the center axis.

According to the embodiments of the present disclosure, the drug delivery tube device is provided with a heat insulating layer to reduce a degree of thermal expansion and thermal contraction of the capillary flow path due to the temperature change of the outside air. As a result, the flow rate of the drug solution flowing through the drug delivery tube device can be kept relatively constant.

According to embodiments of the present disclosure, it is possible to improve the wearing comfort of a patient when the drug delivery tube device is fixed to the patient.

### BRIEF DESCRIPTION OF THE DRAWINGS

FIG. 1 is a perspective view of a drug delivery tube device set according to an embodiment of the present disclosure.
FIG. 2 is a perspective view of the drug delivery tube device set with an attachment member of FIG. 1 removed, as viewed from another angle.
FIG. 3 is a cross-sectional view of the drug delivery tube device set taken along line S1-S1' in FIG. 1.
FIG. 4 is an exploded perspective view of the drug delivery tube device set of FIG. 1.
FIG. 5 is a perspective view of a cover for the drug delivery tube device of FIG. 1.
FIG. 6 is an elevation of the lower surface of the cover for the drug delivery tube device of FIG. 1.
FIG. 7 is a cross-sectional view of the cover taken along line S2-S2' in FIG. 6.
FIG. 8 is a cross-sectional view of the cover taken along line S3-S3' in FIG. 6.
FIG. 9 is a cross-sectional view of the cover taken along line S4-S4' in FIG. 6.
FIG. 10 is a perspective view illustrating an exemplary use state of the drug delivery tube device set of FIG. 1.

### DETAILED DESCRIPTION

Embodiments of the present disclosure are illustrated for the purpose of explaining the technical idea of the present disclosure. The scope of the rights according to the present disclosure is not limited to the embodiments presented below or the detailed descriptions of such embodiments.

All technical and scientific terms used in the present disclosure have the meaning generally understood by those of ordinary skill in the art to which the present disclosure pertains, unless otherwise defined. All terms used in the present disclosure are chosen for the purpose of more clearly describing the present disclosure and are not chosen to limit the scope of rights according to the present disclosure.

As used in the present disclosure, expressions such as "comprising," "including," "having," and the like are to be understood as open-ended terms having the possibility of encompassing other embodiments, unless otherwise mentioned in the phrase or sentence containing such expressions.

The singular form described in the present disclosure may include a plural meaning, unless otherwise mentioned. This applies equally to the singular form recited in the claims.

The terms "first," "second," etc. used herein are used to distinguish a plurality of components from one another, and are not intended to limit the order or importance of the relevant components.

In the present disclosure, when it is mentioned in the present disclosure that one element is "connected" or "coupled" to another element, it is to be understood that said one element may be directly connected or coupled to another element, or may be connected or coupled to another element via a new additional element.

As used in the present disclosure, the term "drug solution" should be understood to include not only a liquid containing a therapeutic substance, but also a liquid that can assist a therapeutic substance or can be used together with a therapeutic substance, and a liquid that can be injected into a patient.

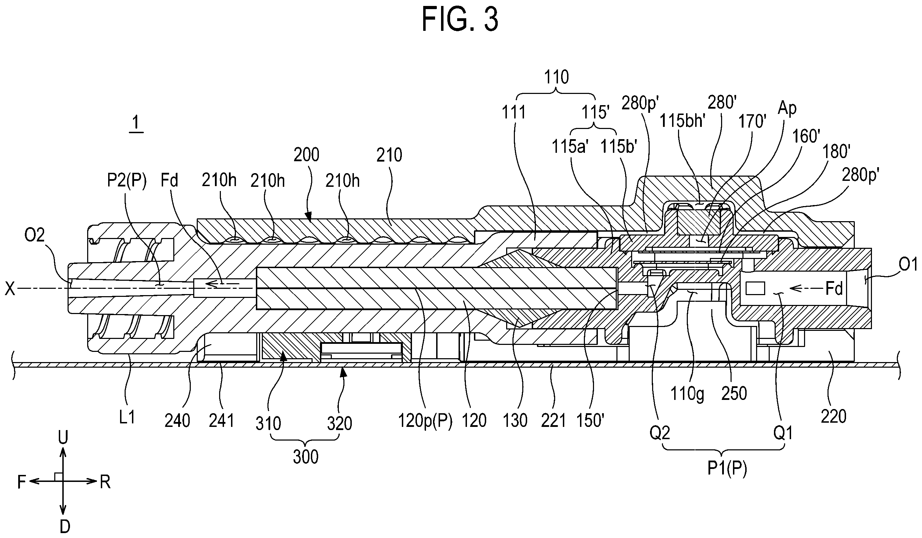

As used in the present disclosure, the terms "upstream" and "downstream" are defined based on the direction in which a drug solution flows through a flow path P from an inlet port O1 to an outlet port O2. The direction of arrow Fd in FIG. 3 is defined as a downstream direction, and the direction opposite to the downstream direction is defined as an upstream direction.

As used in the present disclosure, direction indicators such as "downward," "lower," and the like are based on a direction in which a lower opening 200sa is positioned with respect to an insertion space 200s in the accompanying drawings, and direction indicators such as "upward," "upper," and the like mean the opposite direction. Downward (D) and upward (U) are indicated in the drawings. In addition, as used in the present disclosure, direction indicators such as "front (F)," "rear (R)," "left (Le)," "right (Ri)," and the like are defined as indicated in the drawings. However, a drug delivery tube device set 1 shown in the accompanying drawings may be oriented differently, and the direction indicators may be interpreted accordingly.

Hereinafter, descriptions are made as to embodiments of the present disclosure with reference to the accompanying drawings. In the accompanying drawings, the same or corresponding elements are denoted by the same reference numerals. In the following descriptions of the embodiments, descriptions of the same or corresponding elements may be omitted. However, even if the descriptions of elements are omitted, it is not intended that such elements are not included in a certain embodiment.

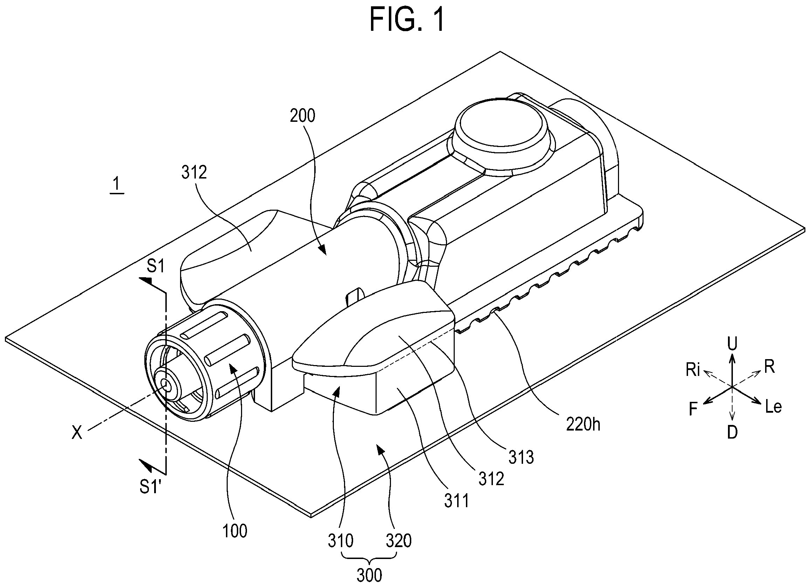

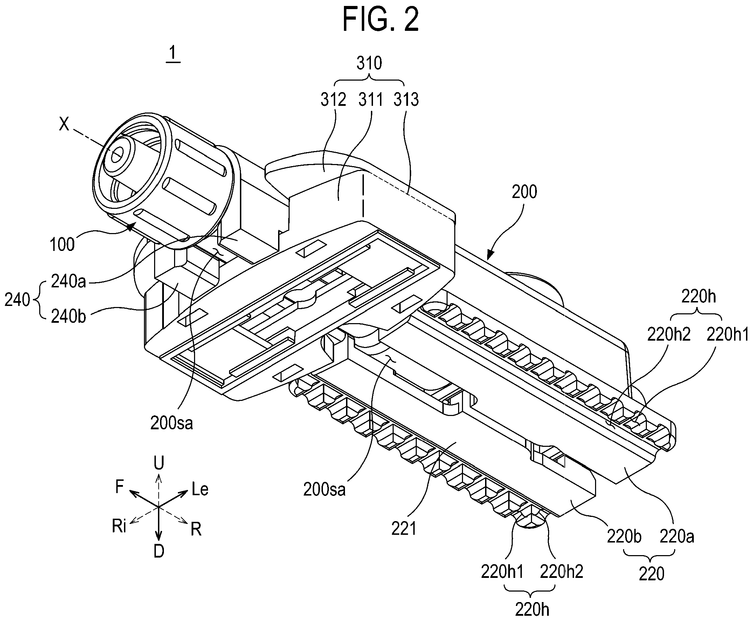

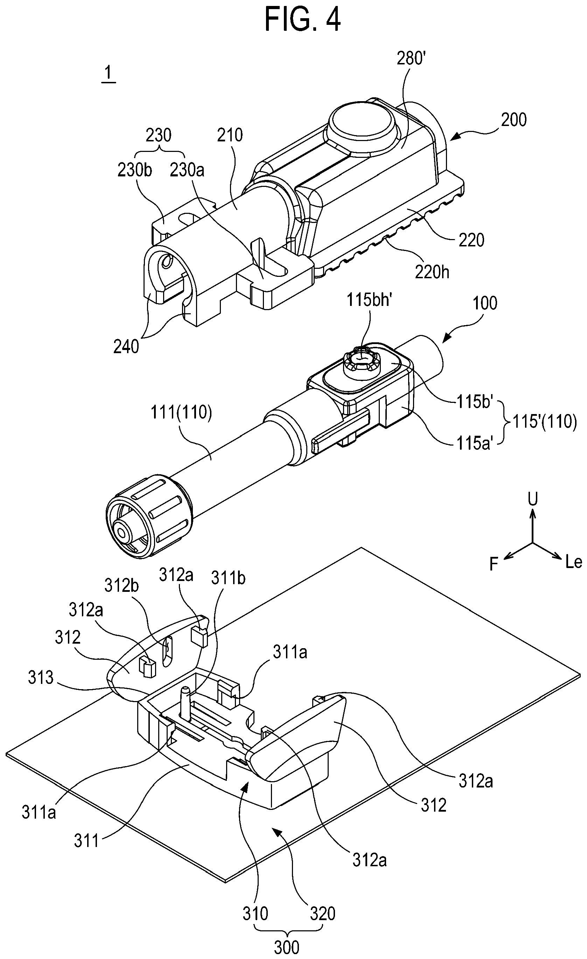

FIG. 1 is a perspective view of a drug delivery tube device set 1 according to an embodiment of the present disclosure. FIG. 2 is a perspective view of the drug delivery tube device set 1 with an attachment member 320 of FIG. 1 removed, as viewed from another angle. FIG. 3 is a cross-sectional view of the drug delivery tube device set 1 taken along line S1-S1' in FIG. 1. FIG. 4 is an exploded perspective view of the drug delivery tube device set 1 of FIG. 1.

Referring to FIGS. 1 to 4, the drug delivery tube device set 1 according to an embodiment of the present disclosure includes a drug delivery tube device 100 having a capillary flow path 120p configured to allow a drug solution to flow therethrough. The drug delivery tube device set 1 includes a cover 200 for the drug delivery tube device that surrounds at least a portion of the outer surface of the drug delivery tube device 100. The drug delivery tube device set 1 may include a fixing device 300 for arranging the cover 200 on a patient's skin.

The drug delivery tube device 100 includes a drug delivery tube 120 having the capillary flow path 120p. The drug delivery tube 120 may limit an hourly flow rate of the drug solution passing through the drug delivery tube device 100. That is, the drug delivery tube 120 may have a function as a flow restricting component. As an example, the drug delivery tube 120 may include a capillary tube. As another example, the drug delivery tube 120 may include a polymer microtube. In addition, the drug delivery tube 120 may be composed of various shapes and materials having a capillary flow path. For example, the capillary flow path 120p may have a diameter of about 0.04 to 0.08 mm, thereby limiting the hourly flow rate of the drug solution.

In one embodiment, the drug delivery tube device 100 includes the drug delivery tube 120 forming the capillary flow path 120p and one or more air passage filters 160' and 170'. In another embodiment not shown, the drug delivery tube device 100 may include the drug delivery tube 120, but may not include the air passage filters 160' and 170'. The following description will be made based on an embodiment in which the drug delivery tube device 100 includes one or more air passage filters 160' and 170'. However, the present disclosure is not limited thereto.

The cover 200 for the drug delivery tube device defines an insertion space 200s having an opening 200sa on the lower side thereof so that the drug delivery tube device 100 can be inserted into the opening 200sa. The cover 200 extends along the circumference of the insertion space 200s around a center axis X perpendicular to the upper-lower direction. The center axis X is an imaginary axis for convenience of description. In the present embodiment, the extension direction of the center axis X may be defined as a front-rear direction. In addition, the direction going away from the center axis X may be defined as a "radial outward direction," and the direction going toward the center axis X may be defined as a "radial inward direction."

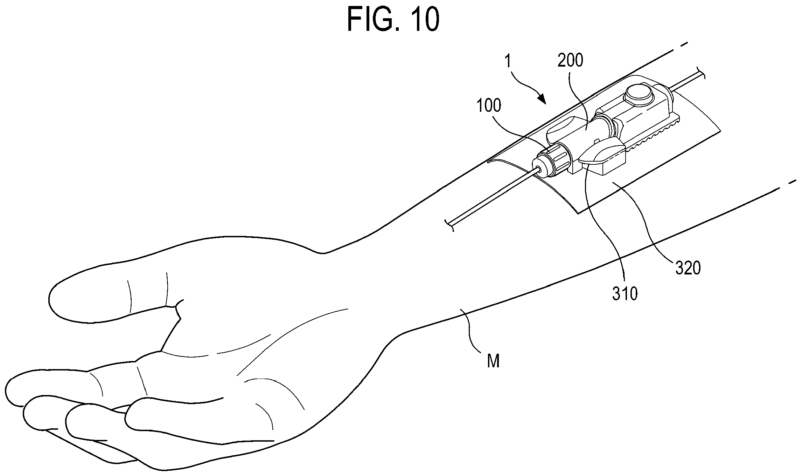

The cover 200 provides a heat insulating layer to the drug delivery tube 120 forming the capillary flow path 120p disposed therein, thereby reducing the amount of a change in cross-sectional area of the capillary flow path 120p according to the change in an external temperature. Thus, the flow rate of the drug solution flowing through the drug delivery tube device 100 can be maintained relatively constant. Furthermore, in one embodiment, by forming the opening of the insertion space 200s in the downward direction facing the patient's skin, the drug delivery tube device inserted into the insertion space 200s can efficiently maintain a constant temperature due to the patient's body temperature (*see* FIG. 10).

The fixing device 300 may be attached to a user. The cover 200 may be fixed to the fixing device 300. The lower portion of the cover 200 may be detachably coupled to the fixing device 300.

The cover 200 includes a base 220 having a lower end surface 221 arranged at the same position in the upper-lower direction as the lowermost end L1 of the drug delivery tube device 100 or at a position lower than the lower end L1 in a state in which the drug delivery tube device 100 is inserted into the insertion space 200s (*see* FIG. 3). This makes it possible to improve the wearing comfort of the patient when the drug delivery tube device 100 is fixed to the patient. In this embodiment, the lower end surface 221 is arranged at a position lower than the lowermost end L1. The cover 200 may include an auxiliary base 240 having a lower end surface 241 arranged at the same position in the upper-lower direction as the lowermost end L1 or at a position lower than the lowermost end L1.

The drug delivery tube device 100 may be used as a component of a drug injection device (not shown) for injecting a drug into a patient or a component connected to the drug injection device. By disposing the drug delivery tube device 100 on a tube (not shown) through which a drug solution flows, the hourly flow rate of the drug solution injected into the patient's body can be kept constant. The drug solution flowing through the tube is introduced into the drug delivery tube device 100 through an inlet port O1 of the drug delivery tube device 100. The introduced drug solution passes through the capillary flow path 120p and then an outlet port O2. Thereafter, the drug solution is moved into the tube disposed on the downstream side and introduced into the patient's body through an injection needle or the like.

The drug delivery tube device 100 has a flow path P configured to allow a drug solution to flow therethrough. The drug delivery tube device 100 may be connected to the tube. The drug delivery pipe device 100 includes the drug delivery tube 120 having the capillary flow path 120p constituting a part of the flow path P. The capillary flow path 120p is configured to allow the drug solution to flow therethrough. The drug delivery tube device 100 includes a housing 110 in which the drug delivery tube 120 is disposed. The drug delivery tube 120 may be coupled to the housing 110. At least a portion of the drug delivery tube 120 may be in contact with the inner surface of the housing 110. The drug delivery tube 120 may be disposed to pass through a seal member 130. The inlet of the capillary flow path 120p is formed at an upstream end of the drug delivery tube 120. The upstream end of the drug delivery tube 120 may be in contact with the inner surface of the housing 110 or a separate spacer (not shown).

The flow path P is connected to the tube so that the drug solution passing through the tube flows into the flow path P. At least a portion of the flow path P is formed in the housing 110. The housing 110 is provided with an inlet port O1 and an outlet port O2 for the drug solution. The drug delivery tube device 100 has an inlet port O1 for inflow of the drug solution formed at one of both ends of the drug delivery tube device 100 in the extension direction of the center axis X. The drug delivery tube device 100 has an outlet port O2 for outflow of the drug solution. The outlet port O2 may be located on the side opposite to the inlet port O1 in the extension direction of the center axis X. However, the present disclosure is not limited thereto. The flow path P may be formed to connect the inlet port O1 and the outlet port O2.

The flow path P may include an upstream flow path P1 disposed on the upstream side of the capillary flow path 120p on the flow path P. The upstream flow path P1 has a cross-sectional area greater than that of the capillary flow path 120p. The housing 110 may form the upstream flow path P1. The inlet port O1 of the drug delivery tube device 100 is an inlet of the upstream flow path P1. The upstream flow path P1 may connect the inlet port O2 and the capillary flow path 120p.

The flow path P may include a downstream flow path P2 disposed on the downstream side of the capillary flow path 120p on the flow path P. The downstream flow path P2 has a cross-sectional area greater than that of the capillary flow path 120p. The housing 110 may form the downstream flow path P2. The outlet port O2 of the drug delivery tube device 100 is an outlet of the downstream flow path P2. The downstream flow path P2 may connect the capillary flow path 120p and the outlet port O2.

The drug delivery tube device 100 may have an air passage Ap branched from the flow path P and connected to the outside. The air passage Ap is formed to connect the middle of the flow path P and the external space. The air passage Ap includes a vent hole 115bh' formed in the housing 110. The drug delivery tube device 100 may further include one or more hydrophobic air passage filters 160' and 170' for discharging the air in the drug solution to the outside. A primary air passage filter 160' is coupled to the housing 110. The primary air passage filter 160' blocks the passage of the drug solution but allows the air to pass therethrough.

The one or more air passage filters 160' and 170' include the primary air passage filter 160'. The primary air passage filter 160' may be disposed at the boundary between the air passage Ap and the flow path passage P. In the present embodiment, the primary air passage filter 160' is disposed at the boundary between the air passage Ap and the first flow path Q1. However, the present disclosure is not limited thereto.

The one or more air passage filters 160' and 170' may further include a secondary air passage filter 170'. The secondary air passage filter 170' is disposed on the air passage Ap and configured to allow the air passing through the primary air passage filter 160' to pass therethrough. The secondary air passage filter 170' may perform a function of preventing the internal drug solution from flowing out even when the pores or the adhesive portion of the primary air passage filter 160' is damaged.

The secondary air passage filter 170' may be formed of the same material as the primary air passage filter 160' or may be formed by processing a porous plastic material. For example, the secondary air passage filter 170' may be formed by processing a hydrophobic porous plastic resin material. For example, the material of the secondary air passage filter 170' is available from Porex Corporation at Fairburn, GA 30213, U.S.A. (website: www.porex.com). A product available under the name of Porex Hydrophobic Vents of the Porex Corporation may be used. This product is made of polytetrafluoroethylene.

The drug delivery tube device 100 may further include a hydrophilic boundary filter 180' disposed on the flow path P. The boundary filter 180' may be disposed at the boundary between the first flow path Q1 on the upstream side and the second flow path Q2 on the downstream side on the upstream flow path P1. The boundary filter 180' is configured to act as a pressure interface between the first flow path Q1 and the second flow path Q2 when the boundary filter 180' is wetted with the drug solution. The first flow path Q1 and the second flow path Q2 may have different internal pressures due to the boundary filter 180'. For example, the boundary filter 180' may be configured to have at least one of a mesh structure and a fiber structure. The boundary filter 180' may additionally have a function of filtering impurities.

The drug delivery tube device 100 may include an inlet filter 150' disposed upstream of the capillary flow path 120p. The inlet filter 150' may be disposed downstream of the primary air passage filter 160'. The inlet filter 150' may be disposed downstream of the boundary filter 180'. The inlet filter 150' is disposed so that the drug solution flowing through the flow path P passes through the inlet filter 150'. The inlet filter 150' may prevent relatively large air bubbles passing through the upstream flow path P1 from blocking the inlet of the capillary flow path 120p.

For example, the inlet filter 150' may be configured to have at least one of a mesh structure and a dense fiber structure. The mesh structure and the dense fiber structure may allow the drug solution to avoid air bubbles blocking the inlet and may allow the drug solution to be absorbed by the inlet filter 150', so that the drug solution flows into the inlet of the drug delivery tube. The mesh structure may also serve to crush large air bubbles. The inlet filter 150' having a mesh structure may be made of a hydrophobic material or a hydrophilic material.

For example, the inlet filter 150' may be a filter made of a hydrophilic material. The inlet filter 150' made of the hydrophilic material may break air bubbles existing upstream of the capillary flow path 120p to prevent the air bubbles from blocking the inlet of the capillary flow path 120p. Furthermore, when the inlet filter 150' made of the hydrophilic material is provided, even in a state in which air bubbles are caught on the upstream side of the inlet filter 150', the drug solution may seep into the inlet filter 150' and may pass through the inlet filter 150'.

As another example, an inlet filter 860 may be a filter made of a hydrophobic material having a relatively coarse mesh that allows the drug solution to pass therethrough. In this case, the inlet filter 150' made of the hydrophobic material may break air bubbles existing upstream of the capillary flow path 120p to prevent the air bubbles from blocking the inlet of the capillary flow path 120p.

The drug delivery tube device 100 includes the housing 110. The housing 110 may be formed by combining two or more components with each other, or may be formed of one component.

The housing 110 includes a delivery tube housing 111 to which the drug delivery tube 120 is coupled. The housing 110 may further include a filter housing 115' to which the one or more air passage filters 160' and 170' are coupled. The filter housing 115' may be coupled to the delivery tube housing 111.

The filter housing 115' may include a filter body 115a' forming at least a portion of the first flow path Q1 and at least a portion of the second flow path Q2. The boundary filter 180' may be disposed on the filter body 115a'. A vent cap 115b' may be coupled to the upper side of the filter body 115a'. The filter body 115a' may accommodate at least a portion of the drug delivery tube 120. The upstream portion of the drug delivery tube 120 is accommodated in the filter body 115a'.

A groove 110g recessed upward may be formed on the lower surface of the housing 110. In the present embodiment, the groove 110g is formed in the filter body 115a. However, the present disclosure is not limited thereto. An insertion portion 250 of the cover 200, which will be described later, may be inserted into the groove 110g.

The filter housing 115' includes a vent cap 115b' coupled to the filter body 115a'. The filter housing 115' defines at least one vent hole 115bh' located at a point where the air passage Ap is connected to the external space. The vent hole 115bh' is formed in the vent cap 115b'. The vent hole 115bh' may be opened upward.

The cover 200 for the drug delivery tube device defines the insertion space 200s into which the drug delivery tube device 100 is inserted. The insertion space 200s has an opening 200sa facing downward. In this embodiment, the insertion space 200s has an opening 200sa on the lower side of the periphery around a center axis X.

The drug delivery tube device 100 includes a cover body 210 and 280' defining the insertion space 200s. In one embodiment, the cover body 210 and 280' includes a capillary flow path accommodation portion 210 for accommodating the capillary flow path 120p therein and a filter accommodation portion 280' for accommodating the air passage filters 160' and 170' therein. In another embodiment, the cover body may include the capillary flow path accommodation portion 210 but may not include the filter accommodation portion 280'. In the present embodiment, description will be made based on the cover body 210 and 280' including the capillary flow path accommodation portion 210 and the filter accommodation portion 280'. However, the present disclosure is not limited thereto.

The drug delivery tube device 100 may include the base 220 disposed below the cover body 210 and 280'. The base 220 extends in a horizontal direction perpendicular to the upper-lower direction. The base 220 has the lower end surface 221 that constitutes the lowermost end of the cover 200. The drug delivery tube device 100 may include a pair of bases 220a and 220b each having the lower end surface 221 that constitutes the lowermost end of the cover 200. The bases 220a and 220b may be disposed left and right with the lower opening 220sa interposed therebetween. The lower end surfaces 221 of the bases 220a and 220b may be disposed at the same level in the upper-lower direction. This makes it possible to improve the wearing comfort of the patient when the cover 200 is fixed to the patient's skin.

The drug delivery tube device 100 may include an engaging portion 230 extending from the cover body 210 and 280' in a horizontal direction perpendicular to the upper-lower direction. In this embodiment, the engaging portion 230 protrudes in the horizontal direction from the filter accommodation portion 280'. However, in another embodiment not shown, the engaging portion may protrude horizontally from the capillary flow path accommodation portion 210. The drug delivery tube device 100 may include a pair of engaging portions 230a and 230b. The engaging portions 230a and 230b may be disposed left and right with the lower opening 220sa interposed therebetween.

The drug delivery tube device 100 may include the auxiliary base 240 disposed below the cover body 210 and 280' and spaced apart from the base 220. The auxiliary base 240 may have the lower end surface 241 that constitutes the lowermost end of the cover 200. The lower end surface 221 of the base 220 and the lower end surface 241 of the auxiliary base 240 may be disposed at the same level in the upper-lower direction. This makes it possible to improve the wearing comfort of the patient when the cover 200 is fixed to the patient's skin. The drug delivery tube device 100 may include a pair of auxiliary bases 240a and 240b. The auxiliary bases 240a and 240b may be disposed left and right with the lower opening 220sa interposed therebetween.

The fixing device 300 may include a fixing member 310 with which the engaging portion 230 is engaged. The fixing member 310 may be configured such that the engaging portion 230 is detachably coupled to the fixing member 310.

The fixing member 310 may include a fixing base 311 fixed to an attachment member 320. The fixing base 311 may be fixed to the upper surface of the attachment member 320.

The fixing member 310 may include a locking portion 312 disposed on the fixing base 311 so as to be rotatable about a hinge portion 313. A pair of locking portions 312 corresponding to the pair of engaging portions 230a and 230b may be provided. The hinge portion 313 provides a rotation axis between the fixing base 311 and the locking portion 312. The rotation axis may be parallel to the center axis X.

One of the fixing base 311 and the locking portion 312 includes a hook portion 312a, and the other includes a hook engaging portion 311a on which the hook portion 312a is engaged. In this embodiment, the locking portion 312 includes the hook portion 312a, and the fixing base 311 includes the hook engaging portion 311a.

In a state in which the hook portion 312a is coupled to the hook engaging portion 311a, the engaging portion 230 of the cover 200 is fixed to the fixing member 310. At this time, the locking portion 312 covers the upper surface of the engaging portion 230.

In a state in which the hook portion 312a is separated from the hook engaging portion 311a, the engaging portion 230 of the cover 200 may be separated from the fixing member 310. By rotating and opening the locking portion 312 about the hinge portion 313, the engaging portion 230 of the cover 200 may be separated from the fixing member 310.

The fixing member 310 may include an insertion protrusion 311b inserted into a hole 230h of the engaging portion 230. The insertion protrusion 311b may protrude upward. The insertion protrusion 311b may protrude from the fixing base 311. A hole or groove 312b, into which the upper end of the insertion protrusion 311b passing through the hole 230h is inserted, may be formed in the lower surface of the locking portion 312.

The fixing device 300 may include the attachment member 320 to which the fixing member 310 is fixed. The attachment member 320 may be configured to be attachable to a user. For example, the attachment member 321 may be formed of a flexible member including a lower surface attached to the patient's skin. In another embodiment not shown, the attachment member may include a strap or a member worn by a user.

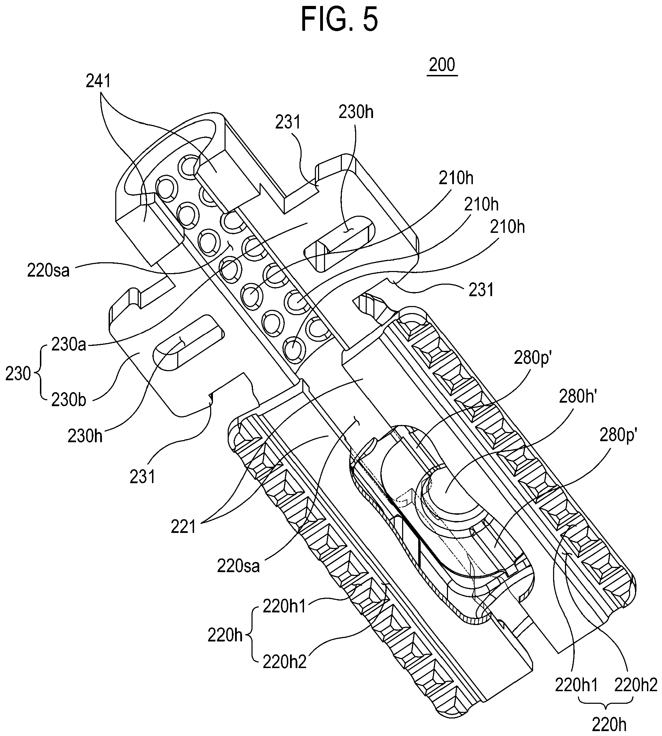

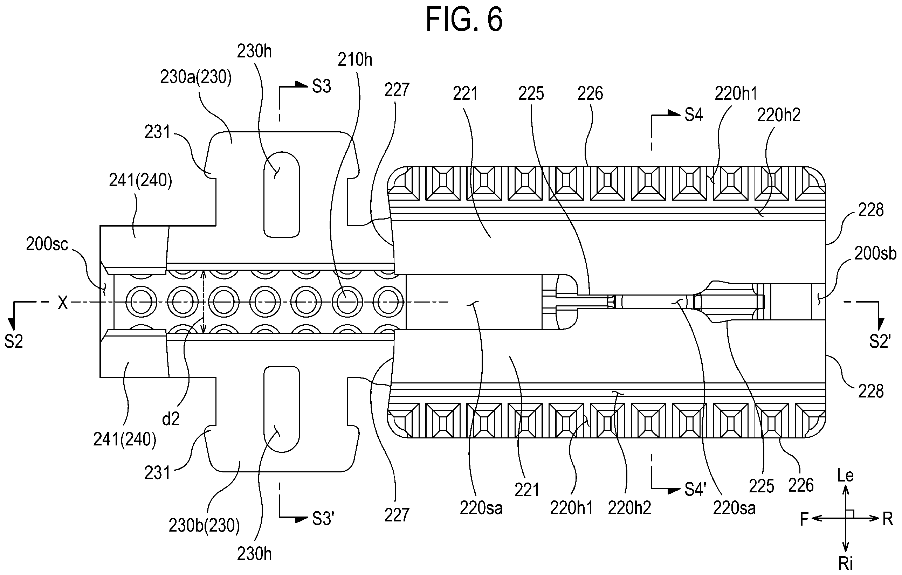

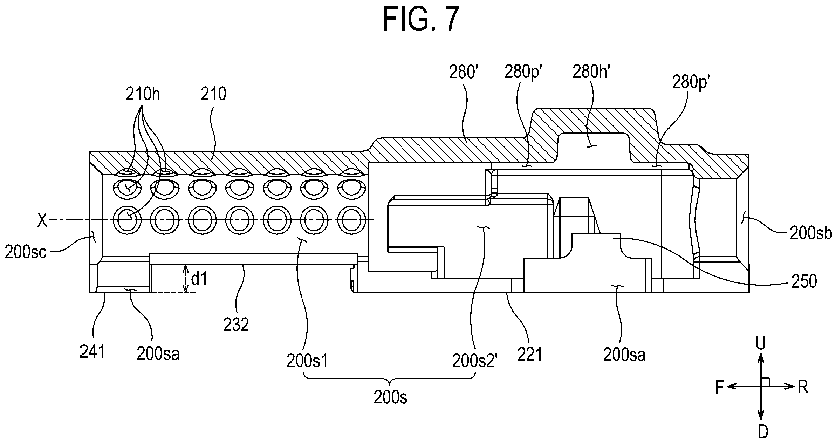

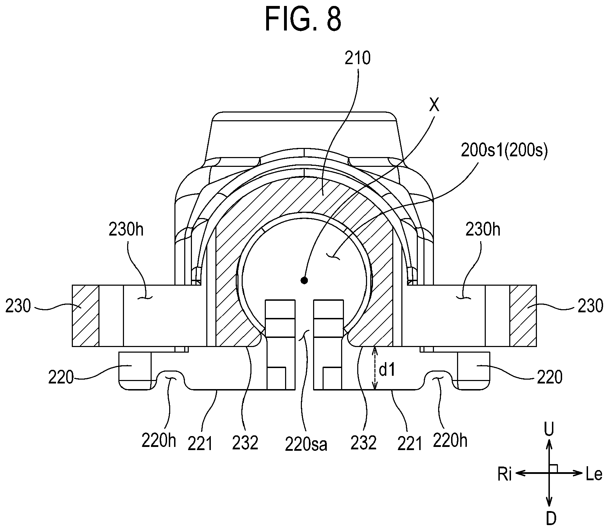

FIG. 5 is a partially cutaway perspective view of the cover 200 for the drug delivery tube device of FIG. 1. FIG. 6 is an elevation of the lower surface of the cover 200 for the drug delivery tube device of FIG. 1. FIG. 7 is a cross-sectional view of the cover 200 taken along line S2-S2' in FIG. 6. FIG. 8 is a cross-sectional view of the cover 200 taken along line S3-S3' in FIG. 7.

The cover 200 for the drug delivery tube device according to an embodiment of the present disclosure defines the insertion space 200s into which the drug delivery tube device 100 having the capillary flow path 120p configured to allow a drug solution to flow is inserted. The insertion space 200s has an opening 220sa formed at the lower side thereof so that the drug delivery tube device 100 can be inserted into the opening 220sa.

The insertion space 200s may extend along the center axis X. The insertion space 200s may be formed in a shape corresponding to a portion of the outer shape of the drug delivery tube device 100.

The insertion space 200s includes a capillary flow path accommodation space 200s1 capable of accommodating the capillary flow path 120p of the drug delivery tube device 100 therein. The capillary flow path accommodation space 200s1 may be formed in a cylindrical shape so as to extend in the front-rear direction as a whole and have an opening 220sa at the lower side thereof. The delivery tube housing 111 may be accommodated in the capillary flow path accommodation space 200s1.

The insertion space 200s may further include a filter accommodation space 200s2' capable of accommodating the one or more air passage filters 160' and 170' of the drug delivery tube device 100 therein. The filter accommodation space 200s2' may extend in the front-rear direction and may be connected to the capillary flow path accommodation space 200s1. The filter accommodation space 200s2' may be disposed behind the capillary flow path accommodation space 200s1. The filter housing 115' may be accommodated in the filter accommodation space 200s2'.

The lower opening 220sa may extend along the center axis X. The lower opening 220sa may have a width d2 in the left-right direction. The width d2 in the left-right direction of the lower opening 220sa may be different depending on the location.

The insertion space 200s may have openings 200sb and 200sc at at least one of both ends in the extension direction of the center axis X. The insertion space 200s may have an upstream opening 200sb at one of both ends in the extension direction of the center axis X on the side on which the inlet port O1 is disposed. The insertion space 200s may have a downstream opening 200sc at one of both ends in the extension direction of the center axis X on the side on which the outlet port O2 is disposed. The upstream opening 200sb and the downstream opening 200sc may be located at both ends of the insertion space 200s.

The cover 200 includes the cover body 210 and 280' extending along the circumference of the insertion space 200s about the center axis X. The inner surface of the cover body 210 and 280' defines the insertion space 200s. The inner surface of the cover body 210 and 280' may face the outer surface of the drug delivery tube device 100.

The cover body 210 and 280' may be formed of a material that is more flexible than the housing 110 of the drug delivery tube device 100. The cover body 210 and 280' may be formed of a flexible material including silicon. For example, the cover body 210 and 280' may be formed of silicon. This makes it possible to improve the thermal insulation performance of the drug delivery tube 120 and the patient's device wearing comfort.

The cover body 210 and 280' may be elastically deformed so that the lower opening 200sa can be opened left and right. The user may widen the lower opening 200sa to insert the drug delivery tube device 100 into the insertion space 200s or to remove the drug delivery tube device 100 from the insertion space 200s.

The cover body 210 and 280' includes the capillary flow path accommodation portion 210 defining the capillary flow path accommodation space 200s 1 of the insertion space 200s. The cover body 210 and 280' may further include the filter accommodation portion 280' defining a filter accommodation space 200s2' of the insertion spaces 200s. In this embodiment, the cover body 210 and 280' includes both the capillary flow path accommodation portion 210 and the filter accommodation portion 280'. In another embodiment not shown, the cover body may include the capillary flow path accommodation portion 210 but may not include the filter accommodation portion 280'. The filter accommodation portion 280' extends rearward from the capillary flow path accommodation portion 210.

A plurality of grooves 210h recessed and spaced apart from one another may be formed on inner surfaces of the cover body 210 and 280'. The grooves 210h may be formed on the inner surface of the capillary flow path accommodation portion 210. The grooves 210h may be arranged to be spaced apart from one another in the extension direction of the center axis X. The grooves 210h may be arranged to be spaced apart from one another in the circumferential direction about the center axis X. The heat insulation performance of the cover 200 may be improved by forming an air layer through the use of the grooves 210h.

A vent insertion groove 280h', into which the protruding portion of the vent cap 115b' of the drug delivery tube device 100 is inserted, may be formed on the inner surface of the filter accommodation portion 280'. The vent insertion groove 280h' may be formed by being recessed upwardly. The vent hole 115bh' may be inserted into the vent insertion groove 280h'.

A flow path groove 280p' extending in a recessed state may be formed on the inner surface of the filter accommodation portion 280'. The flow path groove 280p' may provide a space so that the air leaked to the outside of the drug delivery tube device 100 through the vent hole 115bh' can be better discharged from the space between the drug delivery tube device 100 and the filter accommodation portion 280'. The flow path groove 280p' may extend along the center axis X. One end of the flow path groove 280p' may be connected to the flow path groove 280p'.

The cover 200 may include the base 220 disposed below the cover body 210 and 280'. The base 220 may extend from the cover body 210 and 280'. The base 220 may extend in the horizontal direction perpendicular to the upper-lower direction. The base 220 may have the lower end surface 221 serving as the lowermost end of the cover 200. The lower end surface 221 is a surface that makes contact with the upper surface of the attachment member 320 to press the patient's skin. The lower end surface 221 may include a horizontal surface. The lower end surfaces 221 of the bases 220 may have the same level in the upper-lower direction.

A ventilation groove 220h recessed upward and extending to the edge of the lower end surface 221 may be formed in the lower end surface 221. The sweat generated from the patient's skin can be smoothly discharged through the ventilation groove 220h, which makes it possible to improve the patient's device wearing comfort. The ventilation groove 220h may extend in the horizontal direction.

The ventilation groove 220h may include a first groove 220h1 recessed upward and extending to an outer edge 226 of the lower end surface 221. Among edges 225, 226, 227 and 228 of the lower end surface 221, the edge making contact with the lower opening 200sa is defined as an inner edge 225, and the edge positioned on the side opposite to the inner edge 225 is defined as the outer edge 226. The first groove 220h1 may extend in the left-right direction.

The ventilation groove 220h may include a second groove 220h2 recessed upward and extending to at least one of a front edge 227 and a rear edge 228 of the lower end surface 221. In the present embodiment, both ends of the second groove 220h2 may extend to the front edge 227 and the rear edge 228. The second groove 220h2 may extend in the front-rear direction. The first groove 220h1 may be connected to the second groove 220h2. The first groove 220h1 may extend from the second groove 220h2 to the outer edge 226.

The ventilation groove 220h may be spaced apart from the lower opening 220sa. The ventilation groove 220h may be horizontally spaced apart from the lower opening 220sa. That is, the ventilation groove 220h may be formed so that the ventilation groove 220h is spaced apart from the inner edge 225 of the lower end surface 221 so as not to be connected to the lower opening 200sa. This makes it possible to maintain the thermal insulation performance by preventing the outside air from flowing into the insertion space while securing a space for ventilation.

The cover 200 may include the engaging portion 230 extending from the cover body 210 and 280' in the horizontal direction perpendicular to the upper-lower direction. The engaging portion 230 may protrude outward from the cover body 210 and 280'. The pair of engaging portions 230a and 230b may protrude in the left-right direction.

The engaging portion 230 may be formed so that the engaging portion 230 can be fixed to the fixing device 300. The engaging portion 230 may form at least one of a hook 231 and the hole 230h. The engaging portion 230 may include the hook 231 protruding forward or rearward. The engaging portion 230 may form the hole 230h extending in the upper-lower direction.

A lower end surface 232 of the engaging portion 230 may be disposed at a higher level in the upper-lower direction than the lower end surface 221 of the base 220. The lower end surface 232 may be disposed at a level higher than the lower end surface 221 by a step height d1. The fixing member 310 of the fixing device 300 may be inserted into the space recessed upward by the step height d1.

The cover 200 may include the auxiliary base 240 disposed below the cover body 210 and 280'. The auxiliary base 240 may be spaced apart from the base 220. The auxiliary base 240 may be spaced apart from the base 220 in the extension direction of the center axis X. The auxiliary base 240 may be disposed to be spaced apart from the base 220 with the engaging portion 230 interposed therebetween. The auxiliary base 240 may be disposed in front of the base 220.

The base 240 may have the lower end surface 241 that constitutes the lowermost end of the cover 200. The lower end surface 241 is a surface that makes contact with the upper surface of the attachment member 320 to press the patient's skin. The lower end surface 241 may include a horizontal surface. The lower end surfaces 241 of the pair of bases 240 may have the same level in the upper-lower direction.

The auxiliary base 240 may have the lower end surface 241 disposed at the same level as the lower end surface 221 of the base 220 in the upper-lower direction. The lower end surface 241 may be spaced apart from the lower end surface 221 in the extension direction of the center axis X. The lower end surface 241 may be disposed in front of the lower end surface 221. The lower end surface 241 may be spaced apart from the lower end surface 221 with the engaging portion 230 interposed therebetween. Through the auxiliary base 240, it is possible to further improve the patient's device wearing comfort.

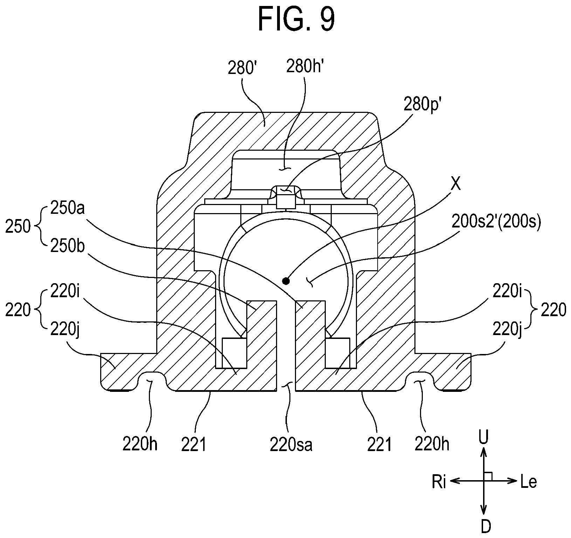

FIG. 9 is a cross-sectional view of the cover 200 taken along line S4-S4' in FIG. 7. Referring to FIG. 9, the base 220 may include the inner protrusion 220i protruding inward from the cover body 210 and 280'. The base 220 may include an outer protrusion 220j protruding outward from the cover body 210 and 280'. The inner protrusion 220i and the outer protrusion 220j may be connected to each other in the left-right direction. The lower end surface of the inner protrusion 220i and the lower end surface of the outer protrusion 220j are connected to each other on the same horizontal plane. The inner protrusion 220i may cover a portion of the lower surface of the drug delivery tube device 100. The length in the front-rear direction of the inner protrusion 220i may be shorter than the length in the front-rear direction of the outer protrusion 220j.

The cover 200 may include an insertion portion 250 protruding toward the inside of the insertion space 200s. The insertion portion 250 may protrude upward. The insertion portion 250 may protrude upward from the inner protrusion 220i of the base 220. The insertion portion 250 may be inserted into the above-described groove 110g formed on the lower surface of the drug delivery tube device 100. The insertion portion 250 may include a pair of insertion portions 250a and 250b corresponding to the pair of bases 220a and 220b.

FIG. 10 is a perspective view illustrating an exemplary use state of the drug delivery tube device set 1 of FIG. 1. The two tubes connected to the inlet port and the outlet port of the drug delivery tube device 100 may be extended so as to be connected to other devices. In FIG. 10, the extension portions except for some sections of the two tubes are omitted.

Referring to FIG. 10, the drug delivery tube device set 1 may be used by being attached to the skin M of the patient. The drug delivery tube device 100 surrounded by the cover 200 may be used in a state in which the lower surface of the attachment member 320 is attached to the patient's skin with the cover 200 fixed to the fixing member 310. Although not shown, by additionally attaching a skin attachment tape to the outside of the cover 200 and the patient's skin, the drug delivery tube device set 1 can be more firmly attached to the patient's skin M. The cover 200 reduces the effect of the temperature of the outside air on the above-described drug delivery tube, thereby helping a drug solution to flow at a constant flow rate through the drug delivery tube device 100. In addition, the base 220 structure of the cover 200 reduces the probability that the patient wearing the drug delivery tube device set 1 feels pain in the skin M, and the ventilation groove 220h helps the sweat generated in the patient's skin M to be discharged through the ventilation groove 220h. This makes it possible to greatly improve the patient's device wearing comfort.

The technical idea of the present disclosure has been described heretofore with reference to some embodiments and examples shown in the accompanying drawings. However, it is to be understood that various substitutions, modifications and alterations may be made without departing from the technical idea and scope of the present disclosure that can be understood by those of ordinary skill in the technical field to which the present disclosure pertains. Further, it is to be understood that such substitutions, modifications and alterations fall within the appended claims.

## Claims

1. A cover for a drug delivery tube device, which is configured to define an insertion space having an opening formed on a lower side thereof so that the drug delivery tube device having a capillary flow path configured to allow a drug solution to flow therethrough is capable of being inserted into the opening, the cover comprising:
a cover body extending along a circumference of the insertion space around a center axis perpendicular to an upper-lower direction.

2. The cover of Claim 1, wherein the cover body is configured to be elastically deformed so that the opening is capable of being widened left and right.

3. The cover of Claim 1, wherein the cover body is formed of a flexible material containing silicon.

4. The cover of Claim 1, wherein a plurality of grooves recessed and spaced apart from one another are formed on an inner surface of the cover body.

5. The cover of Claim 1, further comprising:
a pair of bases disposed below the cover body and extending in a horizontal direction perpendicular to the upper-lower direction, each of the bases having a lower end surface which is a lowermost end of the cover,
wherein the lower end surfaces of the bases are located at the same level in the upper-lower direction.

6. The cover of Claim 5, further comprising:
an auxiliary base disposed below the cover body, the auxiliary base having a lower end surface spaced apart from the lower end surfaces of the pair of bases in an extension direction of the center axis and disposed at the same level in the upper-lower direction with the lower end surfaces of the pair of bases.

7. The cover of Claim 1, further comprising:
a base disposed below the cover body and having a lower end surface which is a lowermost end of the cover,
wherein a ventilation groove recessed upward and extending to an edge of the lower end surface is formed on the lower end surface.

8. The cover of Claim 7, wherein the ventilation groove is spaced apart from the opening.

9. The cover of Claim 1, further comprising:
an engaging portion extending from the cover body in a horizontal direction perpendicular to the upper-lower direction and forming at least one of a hook and a hole.

10. The cover of Claim 1, further comprising:
an engaging portion extending from the cover body in a horizontal direction perpendicular to the upper-lower direction;
a base disposed below the cover body, extending in the horizontal direction perpendicular to the upper-lower direction, and having a lower end surface which is a lowermost end of the cover; and
an auxiliary base disposed below the cover body, spaced apart from the base with the engaging portion interposed between the auxiliary base and the base, and having a lower end surface disposed at the same level in the upper-lower direction as the lower end surface of the base.

11. The cover of Claim 10, wherein the engaging portion has a lower end surface disposed at a higher level in the upper-lower direction than the lower end surface of the base.

12. The cover of Claim 1, wherein the insertion space extends along the center axis.

13. The cover of Claim 1, wherein the insertion space has an opening at at least one of both ends in an extension direction of the center axis.

14. The cover of Claim 1, wherein the cover body includes a capillary flow path accommodation portion configured to form a capillary flow path accommodation space in the insertion space to accommodate the capillary flow path therein, and a filter accommodation portion configured to form a filter accommodation space in the insertion space to accommodate an air passage filter of the drug delivery tube device therein.

15. The cover of Claim 14, wherein a flow path groove recessed and extending along the center axis is formed on an inner surface of the filter accommodation portion.

16. A drug delivery tube device set, comprising:
a drug delivery tube device having a capillary flow path configured to allow a drug solution to flow therethrough; and
a cover configured to define an insertion space having an opening formed on a lower side thereof so that the drug delivery tube device is capable of being inserted into the opening, the cover extending along a circumference of the insertion space around a center axis perpendicular to an upper-lower direction.

17. The drug delivery tube device set of Claim 16, wherein the cover includes a base having a lower end surface disposed at the same position in the upper-lower direction as a lowermost end of the drug delivery tube device or at a position lower than the lowermost end of the drug delivery tube device in a state in which the drug delivery tube device is inserted into the insertion space.

18. The drug delivery tube device set of Claim 16, wherein the cover includes an engaging portion extending in a horizontal direction perpendicular to the upper-lower direction, and
wherein the drug delivery tube device set further comprises:
a fixing member with which the engaging portion is engaged; and
an attachment member to which the fixing member is fixed, the attachment member being configured to be attachable to a user.

19. The drug delivery tube device set of Claim 16, wherein the drug delivery tube device includes a hydrophobic air passage filter for discharging an air in the drug solution to the outside, and
wherein the cover includes a capillary flow path accommodation portion configured to form a capillary flow path accommodation space in the insertion space to accommodate the capillary flow path therein, and a filter accommodation portion configured to form a filter accommodation space in the insertion space to accommodate an air passage filter of the drug delivery tube device therein.

20. The drug delivery tube device set of Claim 16, wherein the drug delivery tube device has an inlet port formed at at least one of both ends in an extension direction of the center axis to allow the drug solution to flow into the inlet port, and the insertion space has an opening disposed at one, at which the inlet port is disposed, of both ends in the extension direction of the center axis.
